(19) Europäisches Patentamt
European Patent Office
Office européen des brevets

(11) Publication number: 0 278 672 A2

(12) **EUROPEAN PATENT APPLICATION**

(21) Application number: 88300922.7

(22) Date of filing: 03.02.88

(51) Int. Cl.4: C07C 127/22 , C07C 157/12 , A01N 47/34

(30) Priority: 10.02.87 JP 28571/87
27.03.87 JP 75609/87
13.08.87 JP 202353/87

(43) Date of publication of application:
17.08.88 Bulletin 88/33

(84) Designated Contracting States:
CH DE FR GB IT LI

(71) Applicant: SUMITOMO CHEMICAL COMPANY, LIMITED
15 Kitahama 5-chome Higashi-ku
Osaka-shi Osaka 541(JP)

(72) Inventor: Mori, Tatsuya
14-7, Mefu-2-chome
Takarazuka-shi(JP)
Inventor: Fujimoto, Hiroaki
14-7, Mefu-2-chome
Takarazuka-shi(JP)
Inventor: Sakamoto, Noriyasu
14-7, Mefu-2-chome
Takarazuka-shi(JP)
Inventor: Fujimoto, Izumi
9-17-374, Minoo-4-chome
Minoo-shi(JP)
Inventor: Ohsumi, Tadashi
7-10-202, Kawazoecho
Nishinomiya-shi(JP)

(74) Representative: Harrison, David Christopher et al
MEWBURN ELLIS & CO 2/3 Cursitor Street
London EC4A 1BQ(GB)

(54) **A benzoylurea derivative and its production and use.**

(57) A benzoylurea derivative useful as an insecticidal and acaricidal composition is represented by the formula,

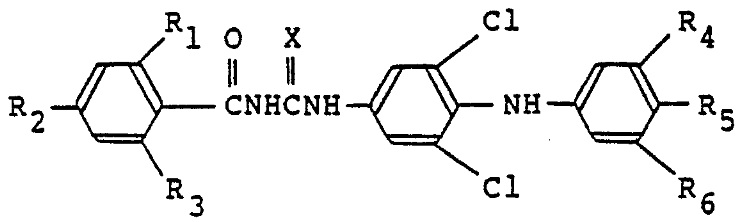

wherein $R_1$ is a chlorine or fluorine atom, $R_2$ and $R_3$, which may be the same or different, are a fluorine or hydrogen atom, at least one of $R_4$, $R_5$ and $R_6$ is a trifluoromethyl group and others, which may be the same or different, are a chlorine, fluorine or hydrogen atom, and X is an oxygen or sulfur atom.

EP 0 278 672 A2

# A BENZOYLUREA DERIVATE AND ITS PRODUCTION AND USE

The present invention relates to a novel benzoylurea derivative, its production and an insecticidal and acaricidal composition containing it as an active ingredient.

Hitherto, benzoylurea compounds belonging to a certain kind are known to have an insecticidal activity and an acaricidal activity (US-A-3989842, EP-A2-72438, JP-A-59-27864 and JP-A-60-51163), and of these compounds there are some which are already on the market like diflubenzuron.

These compounds, however, are insufficient in efficacy or have a problem of manufacturing cost, so that they are not always said to be satisfactory.

In view of the situation like this, the present inventors have made an extensive study about the aniline moiety of the benzoylurea compound, and as a result, have found that a benzoylurea compound derived from a certain kind of 4-anilinoaniline compounds unknown to the literatures has excellent properties: It has a very high insecticidal and acaricidal activity against larvae and nymphs, particularly a high lethal activity against the larvae and nymphs of spider mites and a sterilization activity against the female adults of spider mites, and besides it can be produced at a relatively low cost. The present inventors thus completed the present invention.

The present invention provides a benzoylurea derivative represented by the formula (I) (hereinafter referred to as present compound),

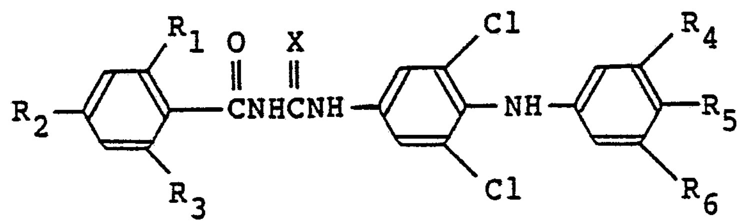

(I)

wherein $R_1$ is a chlorine of fluorine atom, $R_2$ and $R_3$, which may be the same or different, are a fluorine or hydrogen atom, at least one of $R_4$, $R_5$ and $R_6$ is a trifluoromethyl group and others, which may be the same or different, are a chlorine, fluorine or hydrogen atom, and X is an oxygen or sulfur atom,
its production and an insecticidal and acaricidal composition containing it as an active ingredient.

A part of the present compounds is included in the formula described in EP-A2-72438 and JP-A-60-51163, but as is apparent from test examples described later, the present compounds are not only markedly superior particularly in the acaricidal activity, but also have a sterilization activity against spider mites, as compared with the compounds specifically disclosed in said patents and the well-known homologues.

For specific examples of insect pests against which the present compounds are particularly efficacious, there may be given the following: Insects such as larvae of Lepidoptera such as diamond-back moth (Plutella xylostella), rice stem borer (Chilo suppressalis), rice leaf-roller (Cnaphalocrocis medinalis), armyworms and cutworms, etc.; larvae of Diptera such as house mosquitoes (Culex spp.) [e.g. Culex pipiens pallens], Anopheline mosquitoes (Anopheles spp.), Aedes mosquitoes (Aedes spp.), chironomid midges (Chironomidae), houseflies (Muscidae)[e.g. Musca domestica], blow flies (Calliphoridae), flesh flies (Sarcophagidae), tabnid flies (Tabanidae), blackflies (Simuliidae), etc.; nympths of Dictyoptera such as German cockroach (Blattella germanica), etc.; and larvae of Coleoptera; and mites of the family Tetranychidae such as two-spotted spider mite (Tetranychus urticae), carmine spider mite (Tetranychus cinnabarinus), Kanzawa spider mite (Tetranychus kanzawai), citrus red mite (Panonychus citri), European red mite (Panonychus ulmi), etc.; Cheyletidae; Tarsonemidae; Acaridae [e.g. Tyrophagus putrescentiae]; Pyroglyphidae [e.g. Dermatophagoides farinae], etc.

Also, the present compounds are low in toxicity to warm-blooded animals so that it can be orally administered mixed with feeds for animals to domestic animals such as cattle, pigs, horses, sheep, goats, chickens, etc. As a result, the present compounds are excreted from animals as undecomposed, so that the larvae of insect pests living in the excrement of domestic animals [e.g. housefly (Musca domestica), false stablefly (Muscina stabulans), little housefly (Fannia canicularis), blow flies (Calliphoridae), flesh flies (Sarcophagidae), sepsid flies (Sepsidae)], can be exterminated.

Specific examples of the present compound will be shown:

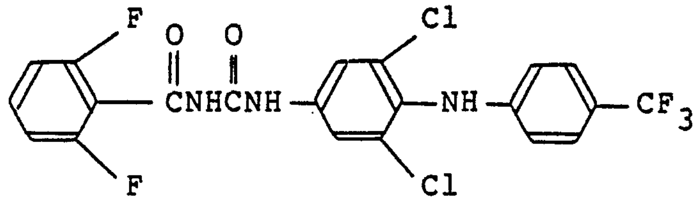

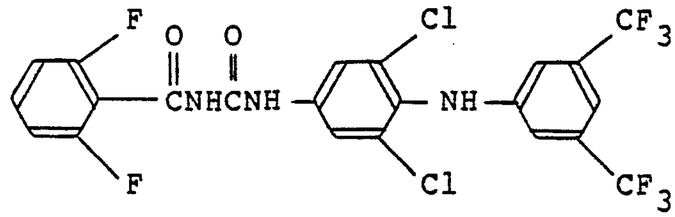

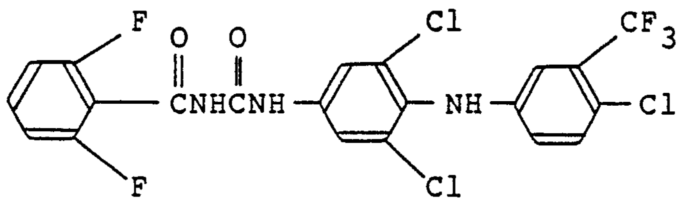

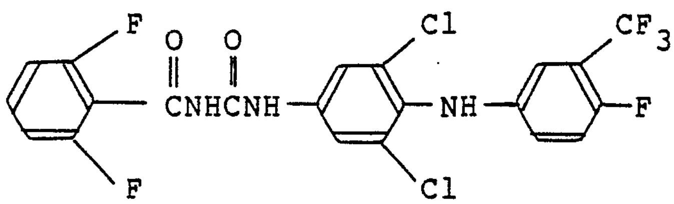

The present compounds can be produced by the following methods.

Method A:

A method of reacting a benzoylisocyanate or benzoylisothiocyanate compound represented by the formula (II),

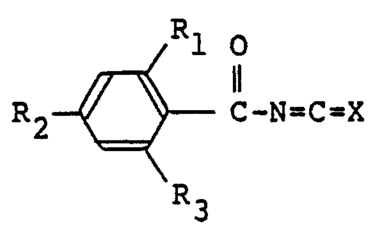

(II)

wherein $R_1$, $R_2$, $R_3$ and X are the same as described above, with a 4-anilinoaniline compound represented by the formula (III),

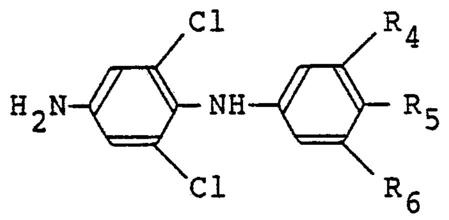

(III)

wherein $R_4$, $R_5$ and $R_6$ are the same as described above.

Method B:

A method of reacting a benzamide compound represented by the formula (IV),

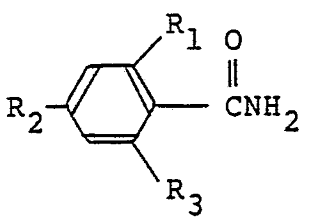

(IV)

wherein $R_1$, $R_2$ and $R_3$ are the same as described above, with an isocyanate or isothiocyanate compound represented by the formula (V),

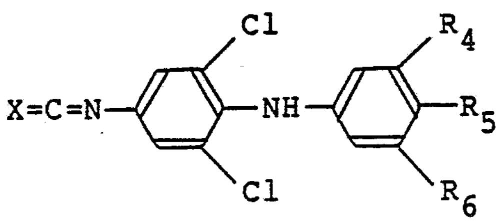

(V)

wherein $R_4$, $R_5$, $R_6$ and X are the same as described above.

In the foregoing Methods A and B, the reaction is usually carried out in the presence of an inert solvent. The solvent usable includes for example hydrocarbons (e.g. benzene, toluene, xylene, chlorobenzene, carbon tetrachloride, chloroform, methylene chloride, 1,2-dichloroethane, nitromethane), ethers (e.g. diethyl ether, tetrahydrofuran, dioxane), ketones (e.g. acetone, methyl ethyl ketone, methyl isobutyl ketone), polar organic solvents (e.g. dimethyl sulfoxide, dimethylformamide, sulfolane) and mixtures thereof.

In Methods A and B, the reaction can generally be carried out under normal pressure, and the object can be attained usually in from 1 to 50 hours. The amount of the compounds which are a raw material are generally in an equimolar ratio, but one of the compounds may be used in excess.

In Methods A and B, the reaction temperature is not particularly limited, but it is in a range of generally from 0° to 80°C, usually from room temperature to 60°C for Method A, and generally from room temperature to 160°C, usually from 80° to 150°C for Method B.

The present compounds thus obtained can be purified if necessary by means such as column chromatography, recrystallization, etc.

The 4-anilinoaniline compound of the formula (III) used in the method of the present invention can be produced, for example, by reacting a 4-halonitrobenzene derivative represented by the formula (VI),

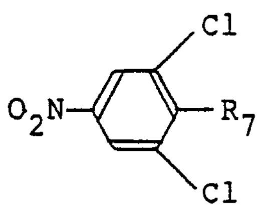

(VI)

wherein $R_7$ is a halogen atom, with an aniline derivative represented by the formula (VII),

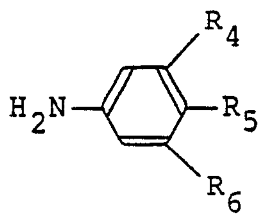

(VII)

wherein $R_4$, $R_5$ and $R_6$ are the same as described above, for example in the presence of a base and preferably in an inert solvent and then reducing the resulting 4-anilinonitrobenzene compound.

Further, said 4-anilinoaniline compound can be converted to an isocyanate or isothiocyanate compound represented by the formula (V) by reacting it with phosgene or thiophosgene according to the usual method.

When the present compounds are used as an active ingredient for insecticidal and acaricidal composition, they may be used as they are without adding any other ingredients. Usually, however, they are formulated into emulsifiable concentrates, wettable powders, dusts, granules, flowable formulations, oil sprays, aerosols, etc. by mixing with solid carriers, liquid carriers, gaseous carriers, surface active agents, other auxiliairies for formulation, baits, etc.

In these preparations, the content of the present compounds, which are an active ingredient, is from 0.01 to 95% by weight. The solid carrier includes for example fine powders or granules of kaolin clay, attapulgite clay, bentonite, terra abla, pyrophyllite, talc, diatomaceous earth, calcite, corn stalk powder, walnut sheel powder, urea, ammonium sulfate, synthetic hydrated silicon dioxide and the like. The liquid carrier includes for example aliphatic hydrocarbons (e.g. kerosene), aromatic hydrocarbons (e.g. benzene, toluene, xylene, methylnaphthalene), halogenated hydrocarbons (e.g. dichloroethane, trichloroethane, carbon tetrachloride), alcohols (e.g. methanol, ethanol, isopropanol, ethylene glycol, cellosolve), ketones (e.g. acetone, methyl ethyl ketone, cyclohexanone, isophorone), ethers (e.g. diethyl ether, dioxane, tetrahydrofuran), esters (e.g. ethyl acetate), nitriles (e.g. acetonitrile, isobutyronitrile), acid amides (e.g. dimethylformamide, dimethylacetamide), dimethyl sulfoxide, vegetable oils (e.g. soybean oil, cotton seed oil) and the like.

The gaseous carrier includes for example freon gas, LPG (liquefied petroleum gas), dimethyl ether and the like. The surface active agent used for emulsification, dispersion, wetting, etc. includes for example anionic surface active agents such as the salt of alkyl sulfates, alkyl(aryl)sulfonates, dialkyl sulfosuccinates, the salt of polyoxyethylene alkylaryl ether phosphoric acid esters, naphthalenesulfonic acid/formalin condensates, etc., and nonionic surface active agents such as polyoxyethylene alkyl ether, polyoxyethylene polyoxypropylene block copolymers, sorbitan fatty acid esters, polyoxyethylene sorbitan fatty acid esters, etc. The auxiliary for formulation such as fixing agents, dispersing agents, etc. includes for example lignosulfonates, alginates, polyvinyl alcohol, gum arabic, molasses, casein, gelatin, CMC (carboxymethyl cellulose), pine oil, agar, etc. The stabilizer includes for example alkyl phosphates [e.g. PAP (isopropyl acid phosphate), TCP (tricresyl phosphate)], vegetable oils, epoxidized oils, the foregoing surface active agents, antioxidants (e.g. BHT, BHA), fatty acid salts (e.g. sodium oleate, calcium stearate), fatty acid esters (e.g. methyl oleate, methyl stearate) and the like.

The preparations thus obtained may be used as they are or diluted with water. Also, they may be used in mixture with other insecticides, acaricides, nematocides, fungicides, herbicides, plant growth regulators, fertilizers, soil improvers, feeds for animals, etc.

When the present compounds are put to practical use as an insecticidal and acaricidal composition, their dosage rate is usually from 1 to 100 g per 10 ares, and their application concentration is from 10 to 500 ppm when emulsifiable concentrates, wettable powders, flowable formulations, etc. are used diluted

with water. Dusts, granules, oil sprays, aerosols, etc. are used as they are without dilution.

The present invention will be illustrated in more detail with reference to the following production examples, reference examples, formulation examples and test examples, but it is not limited to these examples.

Production examples for the present compounds will be shown.

Production example 1 Production of the present compound (5) by Method A

Two hundred and twenty milligrams of 3,5-dichloro-4-[3,5-bis(trifluoromethyl)anilino]aniline was dissolved in 5 ml of toluene, and to the resulting solution was added dropwise a solution of 103 mg of 2,6-difluorobenzoylisocyanate in 5 ml of toluene with stirring and ice-cooling.
After completion of the addition, the reaction solution was stirred overnight at room temperature, and 10 ml of n-hexane was added.
The precipitated crystals were filtered off and dried to obtain 220 mg of N-2,6-difluorobenzoyl-N′-3,5-dichloro-4-[3,5-bis(trifluoromethyl)anilino]phenylurea [present compound (5)] as white crystals.
Yield 68%
m.p. 214.2°C

Production example 2 Production of the present compound (1) by Method B

A mixture of 98 mg of 2,6-difluorobenzamide, 216 mg of 3,5-dichloro-4-[4-(trifluoromethyl)anilino]-phenylisocyanate and 15 ml of xylene was brought into reaction under reflux for 4 hours.
After completion of the reaction, the reaction solution was allowed to cool to room temperature, and 10 ml of n-hexane was added.
The precipitated crystals were filtered off and dried to obtain 260 mg of N-2,6-difluorobenzoyl-N′-3,5-dichloro-4-[4-(trifluoromethyl)anilino]phenylurea [present compound (1)] as white crystals.
Yield 83%
m.p. 207.3°C

Compounds produced by the same methods as above will be shown in Table 1.

Table 1

Compounds represented by the formula:

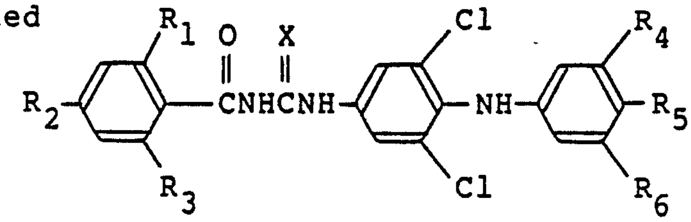

| Compound No. | $R_1$ | $R_2$ | $R_3$ | $R_4$ | $R_5$ | $R_6$ | X | Melting point (°C) |
|---|---|---|---|---|---|---|---|---|
| (1) | F | H | F | H | $CF_3$ | H | O | 207.3 |
| (2) | Cl | H | H | H | $CF_3$ | H | O | 204.4 |
| (3) | F | F | F | H | $CF_3$ | H | O | 197.4 |
| (4) | F | H | F | H | $CF_3$ | H | S | 207.8 |
| (5) | F | H | F | $CF_3$ | H | $CF_3$ | O | 214.2 |
| (6) | Cl | H | H | $CF_3$ | H | $CF_3$ | O | 203.2 |
| (7) | F | F | F | $CF_3$ | H | $CF_3$ | O | 199.3 |
| (8) | F | H | F | $CF_3$ | H | $CF_3$ | S | 182.1 |
| (9) | F | H | F | $CF_3$ | Cl | H | O | 226.2 |
| (10) | Cl | H | H | $CF_3$ | Cl | H | O | 223.3 |
| (11) | F | H | F | $CF_3$ | F | H | O | 208.3 |
| (12) | F | H | F | $CF_3$ | H | H | O | 198.5 |

A production example for a compound used as a material will be shown in a reference example.

Reference example 1

One gram of 3,4,5-trichloronitrobenzene and 0.71 g of 4-(trifluoromethyl)aniline were dissolved in 7 ml of dimethylformamide, and 0.36 g of sodium hydride (as 60% oil suspension) was added in small portions with stirring and ice-cooling. After completion of the addition, stirring was continued overnight at room temperature. The reaction mixture was diluted with 50 ml of diethyl ether, washed successively with 1N

hydrochloric acid and water, dried and concentrated. The resulting crude oily product was purified by thin layer chromatography on silica gel to obtain an oily product. This oily product was dissolved in 7 ml of ethyl acetate, and after adding a catalytic amount of platinum (IV) oxide, stirred at room temperature for 2 hours under hydrogen atmosphere. After removing platinum (IV) oxide from the reaction mixture by filtration, the reaction mixture was concentrated. The resulting crude oily product was purified by thin layer chromatography on silica gel to obtain 0.34 g of 3,5-dichloro-4-[4-(trifluoromethyl)anilino]aniline as white crystals.

Yield 24%

m.p. 154.4°C

$^1$H-NMR ($CDCl_3$):

δ(ppm) 4.3 (2H, br. s)
6.3 (1H, br. s)
6.5 (2H, br. d, J=9.0 Hz)
6.7 (2H, s)
7.3 (2H, br. d, J=9.0 Hz)

Formulation examples will be shown. In the examples, the present compounds are shown by Compound No. in Table 1 and parts are by weight.

Formulation example 1

Ten parts of each of the present compounds (1) to (12), 14 parts of polyoxyethylene styrylphenyl ether, 6 parts of calcium dodecylbenzenesulfonate, 35 parts of xylene and 35 parts of dimethylformamide are well mixed to obtain an emulsifiable concentrate of each compound.

Formulation example 2

Twenty parts of each of the present compounds (1) to (12), 10 parts of fenitrothion, 3 parts of calcium lignosulfonate, 2 parts of sodium lauryl sulfate and 65 parts of synthetic hydrated silicon dioxide are well pulverized and mixed to obtain a wettable powder of each compound.

Formulation example 3

One part of each of the present compounds (1) to (12), 2 parts of carbaryl, 87 parts of kaolin clay and 10 parts of talc are well pulverized and mixed to obtain a dust of each compound.

Formulation example 4

Twenty parts of each of the present compounds (1) to (12), 3 parts of sodium naphthalenesulfonate/formalin condensate and 75 parts of water are well pulverized and mixed, and 2 parts of methyl cellulose is added and mixed as a thickening agent to obtain a flowable formulation of each compound.

Test examples will be shown. The present compounds are shown by Compound No. in Table 1, and compounds used as a control are shown by Compound symbol in Table 2.

Table 2

| Compound symbol | Structural formula | Remark |
|---|---|---|
| (A) | 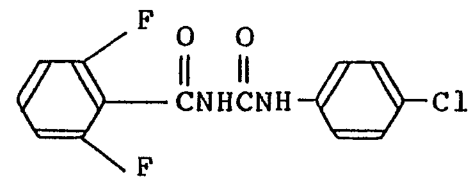 | Diflubenzuron (compound described in US-A-3989842). |
| (B) | 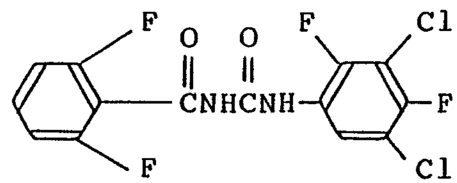 | Teflubenzuron (compound described in JP-A-57-126460). |
| (C) | 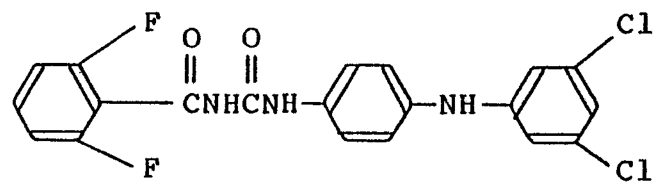 | Compound described in JP-A-60-51163. |
| (D) | 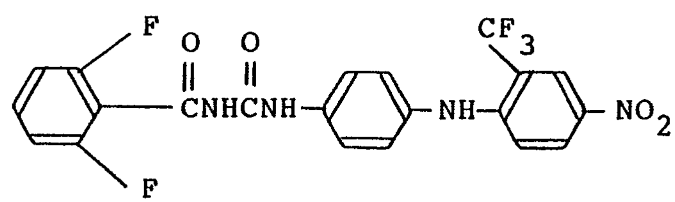 | Same as above. |

- cont'd -

(E) 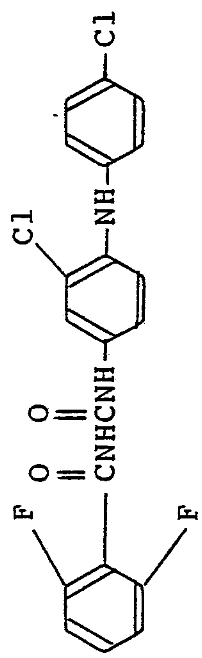 Compound described in EP-A2-72438.

(F) 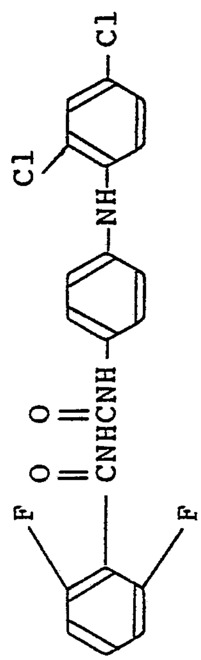 Compound described in JP-A-59-27864.

(G) 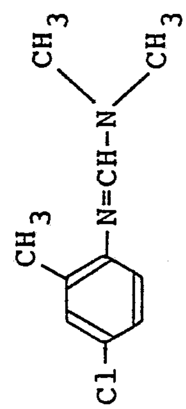 Chlordimeform

Test example 1

The emulsifiable concentrates of the following test compounds obtained according to Formulation example 1 were each diluted with water to a concentration of 0.01 ppm. Thereafter, 100 ml of each dilute solution thus obtained was put in a 180-ml polyethylene cup, and 20 last instar larvae of common mosquito (Culex pipiens pallens) were liberated therein. The larvae were bred on a bait until emergence to obtain an emergence inhibitory ratio (two replications).

The results are shown in Table 3.

Table 3

| Test compound | Emergence inhibitory ratio (%) |
|---|---|
| (1) | 100 |
| (5) | 100 |
| (6) | 100 |
| (7) | 100 |
| (8) | 100 |
| (9) | 100 |
| (11) | 100 |
| (12) | 100 |

Test example 2

The emulsifiable concentrates of the following test compounds obtained according to Formulation example 1 were each diluted with water to a concentration of 50 ppm. Two milliliters of each dilute solution thus obtained was applied onto 13 g of artificial diet for tobacco cutworm (Spodoptera litura) which were then put in a polyethylene cup of 11 cm in diameter. Then, ten fourth instar larvae of tobacco cutworm were liberated in the cup. After six days, the dead and alive were examined to obtain mortality (two replications).

The results are shown in Table 4.

Table 4

| Test compound | Mortality (%) |
|---|---|
| (1) | 100 |
| (2) | 100 |
| (5) | 100 |
| (6) | 100 |
| (7) | 100 |
| (8) | 100 |
| (9) | 100 |
| (11) | 100 |
| (12) | 100 |
| No treatment | 5 |

Test example 3

The emulsifiable concentrates of the following test compounds obtained according to Formulation example 1 were each diluted with water to a pre-determined concentration to obtain a dilute solution of each emulsifiable concentrate. From 20 to 60 deutonymphs of carmine spider mite (Tetranychus cinnabarinus) were transferred onto leaves in a petri dish, and 3 ml of the dilute solution was sprayed thereon. After three days, the number of the adults were counted to obtain an adult emergence inhibitory ratio.

The results are shown in Table 5.

$$\text{Adult emergence inhibitory ratio (\%)} = \left(1 - \frac{\text{adult emergence ratio in the plot treated with chemicals}}{\text{adult emergence ratio in the plot treated with water}}\right) \times 100$$

Table 5

| Test compound | Concentration and adult emergence inhibitory ratio (%) | |
|---|---|---|
| | 500 ppm | 100 ppm |
| (1) | 92 | 81 |
| (2) | 100 | 96 |
| (3) | 100 | 100 |
| (4) | 100 | 100 |
| (5) | 95 | 92 |
| (6) | 90 | 92 |
| (7) | 100 | 96 |
| (8) | 100 | 97 |
| (9) | 100 | 97 |
| (10) | 100 | 90 |
| (11) | - | 95 |
| (12) | - | 87 |
| (A) | 2 | 6 |
| (B) | 13 | 0 |
| (C) | 75 | 4 |
| (D) | - | 39 |
| (E) | 56 | 5 |
| (F) | 13 | 6 |

Test example 4

The emulsifiable concentrates of the following test compounds obtained according to Formulation example 1 were each diluted with water to a concentration of 100 ppm to obtain a dilute solution of each emulsifiable concentrate. Thirty female adults of carmine spider mite (Tetranychus cinnabarinus) were transferred onto a leaf in a petri dish, and 3 ml of the dilute solution was sprayed thereon. After air-drying, 18 female adults were transferred onto untreated leaves in a petri dish, and allowed to oviposit for three days. The eggs obtained were stored in a chamber controlled at 27°C in an artificial weather room. After six days, hatchability of the eggs was examined to obtain a sterilization ratio.

The results are shown in Table 6.

$$\text{Sterilization ratio (\%)} = \left(1 - \frac{\text{hatchability in the plot treated with chemicals}}{\text{hatchability in the plot treated with water}}\right) \times 100$$

Table 6

| Test compound | Sterilization ratio (%) |
|---|---|
| (1) | 98 |
| (2) | 100 |
| (4) | 94 |
| (5) | 98 |
| (6) | 84 |
| (7) | 81 |
| (8) | 77 |
| (9) | 100 |
| (10) | 93 |
| (11) | 99 |
| (12) | 84 |
| (A) | 0 |
| (B) | 0 |
| (C) | 0 |
| (D) | 0 |
| (E) | 0 |
| (F) | 0 |

Test example 5

Ten female adults of carmine spider mite (Tetranychus cinnabarinus) were transferred onto each leaf of potted kidney bean in a primary leaf stage, which had elapsed seven days after sowing, and stored in a constant-temperature room kept at 25°C. After six days, the emulsifiable concentrate of the following test compounds formulated according to Formulation example 1 was diluted with water so that the active ingredient concentration was 500 ppm. Ten milliliters of the dilute solution was sprayed onto the plant on a turn table by a spray gun, and 2 ml of the diluted solution was treated on the pot soil. After 20 days, the degree of damage of each kidney bean by spider mites was examined.

The degree of damage was classified into three stages, -, + and + +.

-: Little damage is observed.
+ : Slight damage is observed.
+ + : Same damage as in the untreated plot is observed.

The results are shown in Table 7.

Table 7

| Test compound | Degree of damage |
|---|---|
| (1) | - |
| (5) | - |
| (6) | - |
| (9) | - ∿ + |
| (G) | ++ |
| No treatment | ++ |

## Claims

1. A benzoylurea derivative represented by the formula,

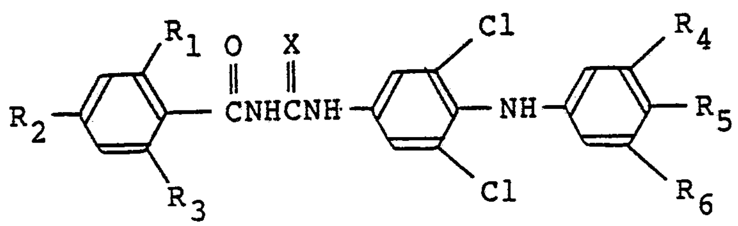

wherein $R_1$ is a chlorine or fluorine atom, $R_2$ and $R_3$, which may be the same or different, are a fluorine or hydrogen atom, at least one of $R_4$, $R_5$ and $R_6$ is a trifluoromethyl group and others, which may be the same or different, are a chlorine, fluorine or hydrogen atom, and X is an oxygen or sulfur atom.

2. The benzoylurea derivative according to Claim 1, wherein $R_5$ is a trifluoromethyl group, and $R_4$ and $R_6$ are a hydrogen atom.

3. The benzoylurea derivative according to Claim 1, wherein $R_4$ and $R_6$ are a trifluoromethyl group, and $R_5$ is a hydrogen atom.

4. The benzoylurea derivative according to Claim 1, wherein $R_4$ is a trifluoromethyl group, $R_5$ is a chlorine or fluorine atom; and $R_6$ is a hydrogen atom.

5. The benzoylurea derivative according to Claim 1 or 2 represented by the formula,

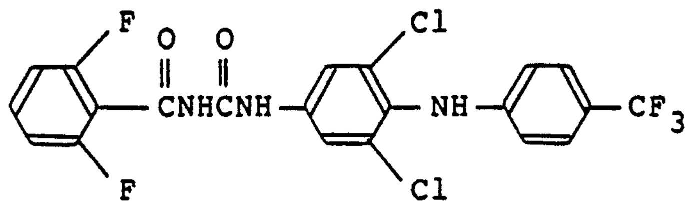

6. The benzoylurea derivative according to Claim 1 or 3 represented by the formula,

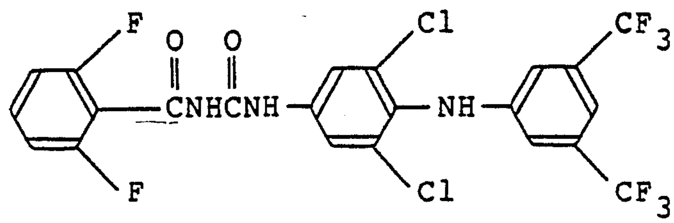

7. The benzoylurea derivative according to Claim 1 or 4 represented by the formula,

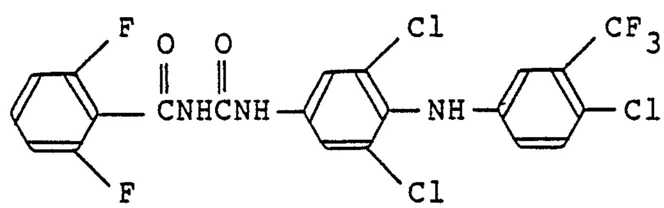

8. A method for producing a benzoylurea derivative represented by the formula,

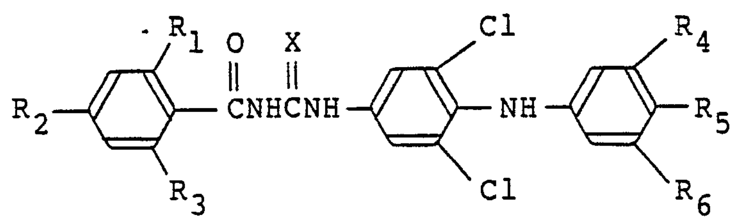

wherein $R_1$ is a chlorine or fluorine atom, $R_2$ and $R_3$, which may be the same or different, are a fluorine or hydrogen atom, at least one of $R_4$, $R_5$ and $R_6$ is a trifluoromethyl group and others, which may be the same or different, are a chlorine, fluorine or hydrogen atom, and X is an oxygen or sulfur atom,
which comprises reacting a benzoylisocyanate or benzoylisothiocyanate compound represented by the formula,

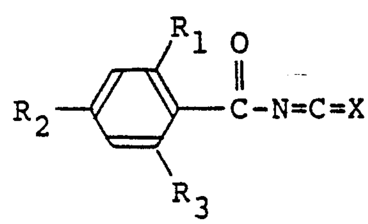

wherein $R_1$, $R_2$, $R_3$ and X are the same as described above, with a 4-anilinoaniline compound represented by the formula,

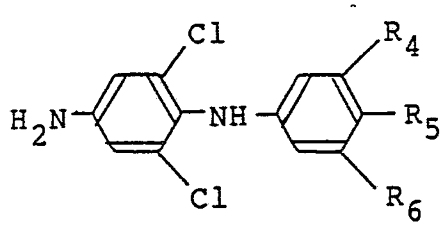

wherein $R_4$, $R_5$ and $R_6$ are the same as described above.

9. A method for producing a benzoylurea derivative represented by the formula,

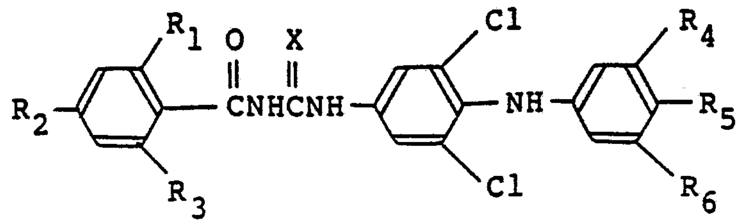

wherein $R_1$ is a chlorine or fluorine atom, $R_2$ and $R_3$, which may be the same or different, are a fluorine or hydrogen atom, at least one of $R_4$, $R_5$ and $R_6$ is a trifluoromethyl group and others, which may be the same or different, are a chlorine, fluorine or hydrogen atom, and X is an oxygen or sulfur atom,
which comprises reacting a benzamide compound represented by the formula,

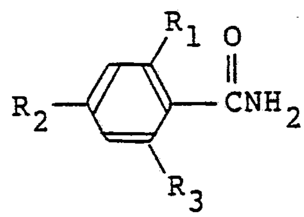

wherein $R_1$, $R_2$ and $R_3$ are the same as described above, with an isocyanate or isothiocyanate compound represented by the formula,

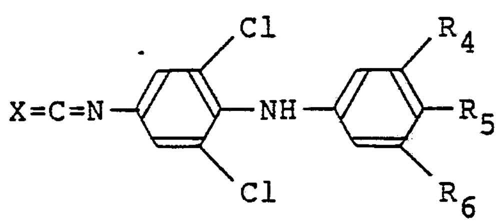

wherein $R_4$, $R_5$, $R_6$ and X are the same as described above.

10. An insecticidal and/or acaricidal composition which comprises as an active ingredient an insecticidally and/or acaricidally effective amount of the compound according to Claim 1, and an inert carrier or diluent.

11. The composition according to Claim 10, wherein $R_5$ is a trifluoromethyl group, and $R_4$ and $R_6$ are a hydrogen atom.

12. The composition according to Claim 10, wherein $R_4$ and $R_6$ are a trifluoromethyl group, and $R_5$ is a hydrogen atom.

13. The composition according to Claim 10, wherein $R_4$ is a trifluoromethyl group, $R_5$ is a chlorine or fluorine atom, and $R_6$ is a hydrogen atom.

14. A method for controlling or exterminating insects and/or acarids which comprises applying as an active ingredient an insecticidally and/or acaricidally effective amount of the compound according to Claim 1 to the locus where insects and/or acarids propagate.

15. Use of the compound according to Claim 1 as an insecticide and/or acaricide.